# EUROPEAN PATENT SPECIFICATION

(11) **EP 1 196 153 B1**
(45) Date of publication and mention of the grant of the patent: **19.11.2003**
(21) Application number: 00947995.7
(22) Date of filing: 25.07.2000
(51) Int. Cl.: A61K 31/00, A61K 31/352, A61K 31/37, A61K 31/5377, A61K 35/52, A61D 19/00, A61D 19/02, A01N 1/02, A61P 15/08

(54) **PROCESS FOR THE IMPROVEMENT OF SPERMATOZOA FERTILIZATION ACTIVITY**
VERFAHREN ZUR VERBESSERUNG DER FERTILISIERENDEN AKTIVITÄT VON SPERMATOZOA
PROCESSUS DESTINE A AMELIORER L'ACTIVITE DE FECONDATION DES SPERMATOZOIDES

(30) Priority: 26.07.1999 IT FI990171
(43) Date of publication of application: 17.04.2002
(60) Divisional application: 03102581.0
(73) Proprietor: Applied Research Systems ARS Holding N.V., Curacao (AN)
(72) Inventor: LUCONI, Michaela, I-50137 Firenze (IT); BALDI, Elisabetta, I-50018 Scandicci-Firenze (IT); FORTI, Gianni, I-50134 Firenze (IT)
(74) Representative: Serono International S.A.
(86) International application number: EP0007108
(87) International publication number: WO01007021

(56) References cited:
- EP-A- 1 057 505
- WO-A-95/29673
- US-A- 4 774 088
- US-A- 5 733 759
- FRASER ET AL.: "Ca2+-regulating mechansims that modulate bull sperm capacitation and acrosomal exocytosis as determeined by Chlortetracycline analysis" MOL. REPROD. DEV., vol. 40, no. 2, 1995, pages 233-241, XP001009962
- NASS-ARDEN ET AL.: "Modulation of mammalian pserm motility by quercetin" MOL. REPROD. DEV., vol. 25, no. 4, April 1990 (1990-04), pages 369-373, XP001009971
- ROLDAN ET AL.: "Is a Ca2+-ATPase involved in Ca2+ regulation during capacitation and the acrosome reaction of guinea-pig spermatozoa ?" J. REPROD. FERTIL., vol. 85, no. 1, January 1989 (1989-01), pages 297-308, XP001009963
- HAMMERSTEDT ET AL.: "Motility, heat, and lactate production in ejaculated bovine sperm" ARCH. BIOCHEM. BIOPHYS., vol. 266, no. 1, October 1988 (1988-10), pages 111-123, XP001009970
- DASGUPTA ET AL.: "A possible role for Ca2+-ATPase in human soerm capacitation" J. REPROD. FERTIL., vol. 102, no. 1, September 1994 (1994-09), pages 107-116, XP001009960
- BREITBART : "Calcium metabolism in spematozoa: a target for contarception" ADV. CONTRACEPTIVE DELIVERY SYSTEMS, vol. 9, no. 2-3, 1993, pages 93-95, XP001009977
- FRASER ET AL.: "Ca2+-related changes in mouse sperm capacitation state: a possible for Ca2+-ATPase" J. REPROD. FERTIL., vol. 96, no. 1, September 1992 (1992-09), pages 363-377, XP001009969
- FISHER ET AL.: "Phosphoinositide 3-kinase is involved in the induction of the human sperm acrosome reaction downstream of tyrosine phosphorylation" MOL. HUMAN REPROD., vol. 4, no. 9, September 1998 (1998-09), pages 849-855, XP001009974
- FENG ET AL.: "The c-kit receptor and its possible signaling transduction pathway in mouse spermatozoa" MOL. REPROD. DEV., vol. 49, no. 3, March 1998 (1998-03), pages 317-326, XP001009976
- BLUME-JENSEN ET AL.: "Kit/stem cell factor receptor-induced activation of phosphatidylinositol 3'-kinase is essential for male fertility" NATURE GENETICS, vol. 24, no. 2, February 2000 (2000-02), pages 157-162, XP001009975
- JACK ET AL.: "Glycogen synthase kinase-3 phosphorylation- Evidence for a role for phosphoinositide-3-kinase signaling pathway in sperm motility regulation" BIOL. REPROD., vol. 62, no. suppl-1, 15 - 18 July 2000, pages 206-207, XP001009990
- ARAVINDAKSHAN ET AL.: "Studies on germinal effects of quercetin, a naturally occuring flavonoid" MUTAT. RES., vol. 144, 1985, pages 99-106, XP001009965
- DATABASE WPI Week 199620 Derwent Publications Ltd., London, GB; AN 1996-195278 XP002170825 & JP 08 066183 A (SHINGIJUTSU JIGYODAN), 12 March 1996 (1996-03-12)
- DATABASE WPI Week 199303 Derwent Publications Ltd., London, GB; AN 1993-024773 XP002170826 & KR 9 202 285 B (PACIFIC CHEM. CO.), 21 March 1992 (1992-03-21)

## Description

### FIELD OF THE INVENTION

The invention relates to a process for the improvement of spermatozoa fertilization activity, in particular for the increase of spermatozoa motility by inhibiting the enzyme phosphatidylinositol-3 kinase (PI3K). The invention further relates to uses and methods of PI3K inhibitors in infertility and assisted reproduction techniques as well as to a medium for storage and/or transportation of spermatozoa comprising PI3K inhibitors.

### BACKGROUND OF THE INVENTION

The infertility of a couple is defined as the inability of the woman to conceive after at least a year of regular unprotected sexual relations. Infertility may be caused by a multitude of factors, in which male factors play a fundamental role in around 40-50% of cases. Reduced male fertility is generally linked to alterations in seminal parameters such as morphology, motility and sperm count.

Various assisted fertilization techniques (ARTs) are proposed as treatment for infertility of the couple, in many cases making it possible to overcome the problem of both male and the female factors. These methods, the choice of which depends on the type of diagnosis made, may involve the collection of male and female gametes (spermatozoa and oozytes). The further treatment varies according to the cause of the infertility. The gametes may be transferred directly into the Fallopian tube (GIFT= gamete intra fallopian transfer) or are brought into contact with each other in a test tube. If the latter leads to fertilization of the oozyte, the resulting zygote or embryo is transferred into the uterus (IVFET = In Vitro Fertilization and Embryo Transfer).

When infertility is due to male factor(s), parameters of the seminal liquid and in particular the count and motility of spermatozoa determine the choice of the particular assisted fertilization method used. In the most serious cases of male-factor infertility the spermatozoa count and/or their motility is very low. The fertilization activity of semen is usually assessed in a spermogram. According to WHO standards, which can be taken from the "WHO manual" (WHO laboratory manual for the examination of human semen and sperm-cervical mucus interactions, 4^{th} edtition, Cambridge University Press 1999), semen are classified into the following groups:
- Normozoospermia: When all the spermatozoal parameters are normal together with normal seminal plasma ,WBCs (White blood cells) and no agglutination;
- Oligozoospermia: When sperm concentration is < 20 million/ml;
- Teratozoospermia: Fewer than 50% spermatozoa with forward progression(categories (a) and (b)) or fewer than 25% spermatozoa with category (a) movement;
- Asthenozoospermia: Fewer than 50% spermatozoa with normal morphology;
- Oligoasthenoteratozoospermia: Signifies disturbance of all the three variables (combination of only two prefixes may also be used);
- Azoospermia: No spermatozoa in the ejaculate.

Normal values of semen parameters have been issued by WHO that are generally used as reference. The fraction of motile sperm in semen is measured either by manual counting or using a computer assisted semen analysis (CASA) system. Motility is assessed at the time of semen liquefaction and after 1 and 3 hours to detect asthenozoospermia. Manual counting classifies sperm cells into 4 categories (immotile, locally motile, non linear and linear motile) using qualitative subjective criteria of selection. Many infertility centers now use CASA systems for objective measurements of sperm motion and positive correlations have been found between motion parameters such as the amplitude of lateral head displacement, curvilinear velocity, linearity and straight-line velocity and fertilization rates *in vitro* but the threshold levels for these motion characteristics have not yet been established to meet a general consensus.

In case of severe male factor infertility, micro-assisted fertilization techniques can be used. Among these techniques, intracytoplasmatic sperm injection (ICSI) is the most common and has the highest percentage of success. However, the safety of the ICSI procedure for the health of the resulting conceptus or embryo is still matter of debate (Edwards, 1999; Luetjens et al., 1999). In addition, ICSI is far more expensive and more time consuming as compared to IVF.

Thus, the possibility to recover a higher number of spermatozoa showing a higher motility could allow several oligoasthenospermic men to enter IVF rather than ICSI programs.

Various methods have attempted at increasing the motility of the spermatozoa, like treatment of spermatozoa with pentoxyphylene, platelet activating factor or progesterone, for instance. However, the results obtained are variable and the responsiveness of the spermatozoa is not predictable.

Therefore, the finding of new methods and agents to improve sperm cell motility, leading to an improvement of the fertilization activity or fertilization rate, is highly desirable and urgently needed.

Phosphatidylinositol-3-kinases (PI3Ks) belong to a family of enzymes involved in signal transduction of tyrosine kinase receptors. Phosphatidylinositol-3-kinases, also called phosphoinositide-3-kinases (PI3Ks) generate lipids which are implicated in receptor-stimulated signalling and in the regulation of membrane traffic. Several distinct classes of PI3Ks have been identified that have been conserved throughout eukaryotic evolution. Potential signalling pathways downstream of PI3Ks have been elucidated and PI3K function is being characterized in several model organisms, as reviewed e.g. by Vanhaesebroeck et al. (1997). PI3Ks are heterodimeric enzymes present in various isoforms and composed of a catalytic subunit of 110 kDa, which is associated with a regulating subunit of 85 kDa.

In somatic cells phosphoinositide-3-kinases (PI3-kinases) are activated upon interaction with both receptor tyrosine kinases (RTK) and G-proteins resulting in the production of moieties involved in the inositol phospholipid signalling pathway. The enzyme is also present and active in human spermatozoa.

Several selective inhibitors of PI3Ks have been described. Wortmannin is one of the most well-known specific inhibitors. Wortmannin and analogs thereof are described, for example, in EP 0 635 268 A1, EP 0 648 492 A2 or EP 0 658 343 A1. These compounds are known to be involved in the treatment of neoplasms, atherosclerosis, and bone disorders. Other phosphatidylinositol-3-kinase inhibitors are 2-(4-morpholinyl)-8-phenyl-4H-1-benzopyran-4-one (LY294002), described in Vlahos et al. (1994 and 1995), related to the bioflavonoid quercetin (Vlahos et al., 1994), for example.

LY294002 has not been described to improve spermatozoa fertilization activity so far or is known to be involved in fertilization.

### SUMMARY OF THE INVENTION

It has now been found in accordance with the invention that an inhibitor of the enzyme phosphatidylinositol-3-kinase can significantly improve the parameters determining sperm cell fertilization activity, in particular the sperm cell motility.

The invention therefore relates to a process for enhancing spermatozoa fertilization activity, in particular for increasing the motility of the spermatozoa, comprising treating the spermatozoa with a phosphatidylinositol-3 kinase (PI3K) inhibitor, which is selected from the group consisting of 2-(4-morpholinyl)-8-phenyl-4H-1-benzopyran-4-one (LY294002), and derivatives and analogues thereof. The invention further relates to spermatozoa in which the activity of the phosphatidylinositol-3 kinase is inhibited, as well as to the use of a PI3K inhibitor, which is selected from the group consisting of 2-(4-morpholinyl)-8-phenyl-4H-1-benzopyran-4-one (LY294002), and derivatives and analogues thereof, for improving the fertilization rate in assisted reproduction techniques (ART). A third aspect of the invention concerns the use of a phosphatidylinositol-3 kinase (PI3K) inhibitor, which is selected from the group consisting of 2-(4-morpholinyl)-8-phenyl-4H-1-benzopyran-4-one (LY294002), and derivatives and analogues thereof, for the manufacture of a medicament for the treatment of infertility, in particular male infertility. A fourth aspect of the present invention relates to methods of ART therapy comprising treating spermatozoa with an phosphatidylinositol-3-kinase inhibitor, which is selected from the group consisting of 2-(4-morpholinyl)-8-phenyl-4H-1-benzopyran-4-one (LY294002), and derivatives and analogues thereof. A fifth aspect of the invention relates to a medium for storage and/or transportation of spermatozoa comprising a phosphatidylinositol-3-kinase inhibitor, which is selected from the group consisting of 2-(4-morpholinyl)-8-phenyl-4H-1-benzopyran-4-one (LY294002), and derivatives and analogues thereof.

### BRIEF DESCRIPTION OF THE DRAWINGS

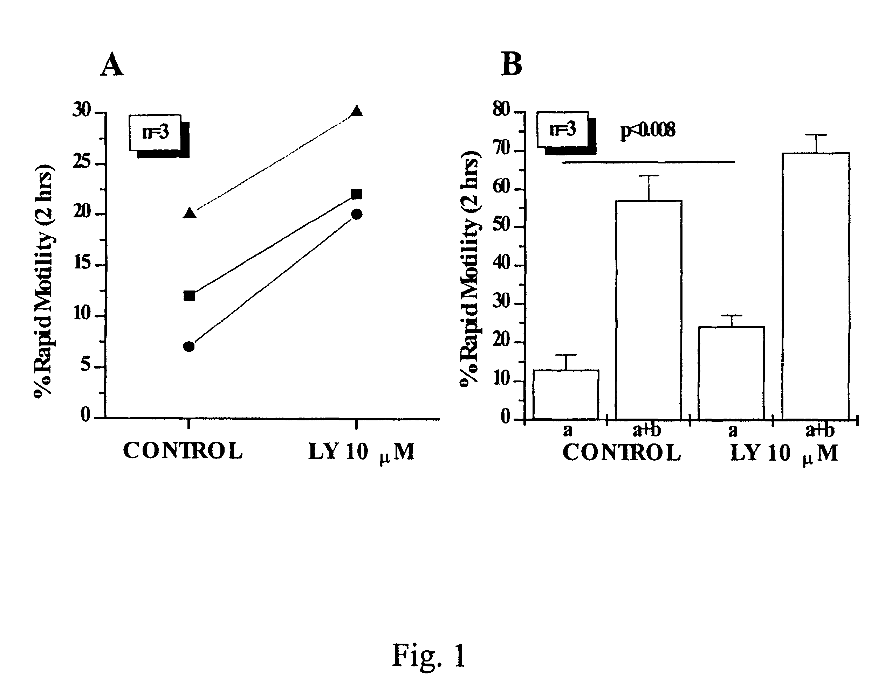

- Fig. 1: shows the effect of 10µM of the PI3K inhibitor LY294002 on the motility of spermatozoa. Fig. 1 A: Rapid progressive component in three individual patients. Each symbol represents one individual patient. Fig. 1 B: Rapid progressive motility (a) and rapid + slow progressive motility (a+b) of the spermatozoa; mean values ± SEM. P indicates the p-value. LY indicates LY294002.

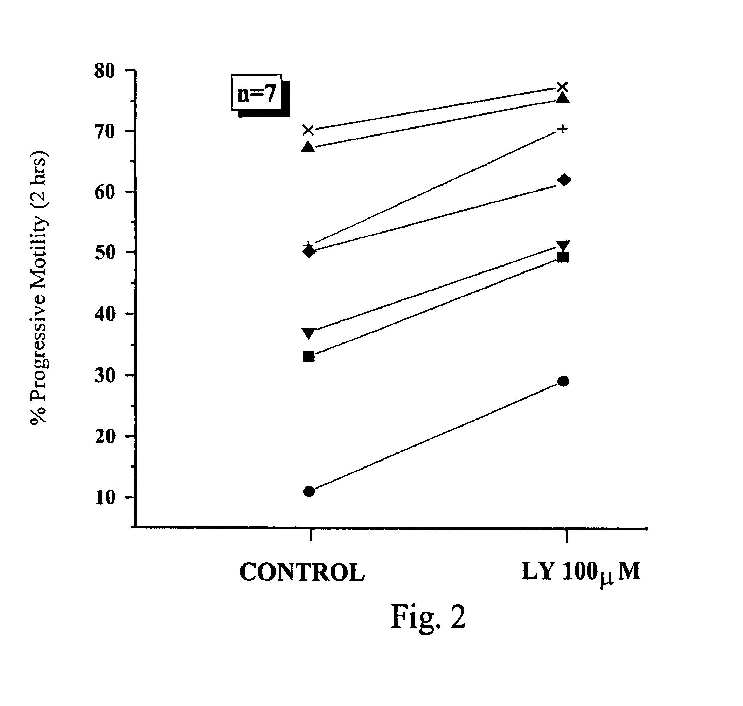

- Fig. 2: shows the effect of 100µM LY294002 on the progressive motility of the spermatozoa obtained by the swim-up method.

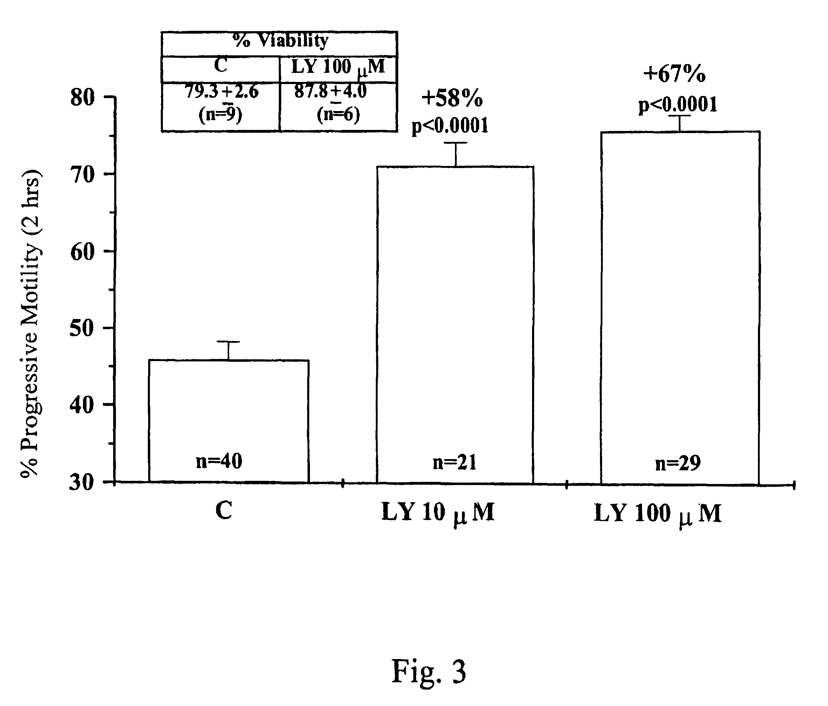

- Fig. 3: shows the effect of 10µM and 100µM LY294002 on the progressive motility of spermatozoa obtained by the swim-up method; mean values ± SEM. C indicates control, LY indicates LY294002. The insert shows the effects of 100µM LY294002 on the vitality of spermatozoa obtained by the swim-up method.

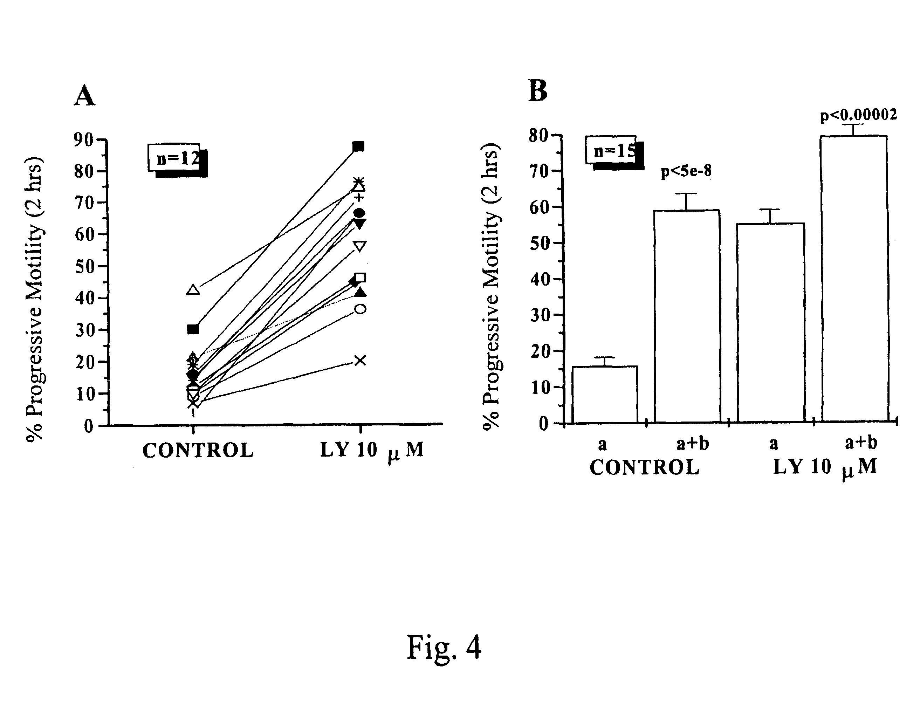

- Fig. 4: shows the effect of 10µM LY294002 on the progressive motility of spermatozoa obtained by the swim-up method. Fig. 4 A shows the effect on the rapid progressive component (motility a) in individual patients. Each symbol indicates an individual patient. Fig. 4 B shows a histogram depicting the effect on the fast progressive motility (a) and the fast plus slow progressive motility (a+b) of spermatozoa (mean values ± SEM).

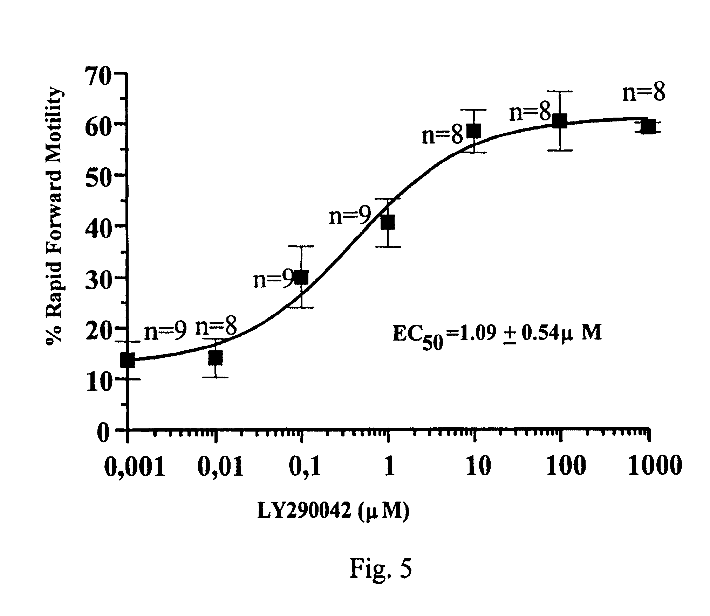

- Fig. 5: shows a dose-response curve of increasing concentrations of LY294002 (µM) (x-axis) on the rapid forward motility of the spermatozoa (y-axis) obtained by the swim-up method.

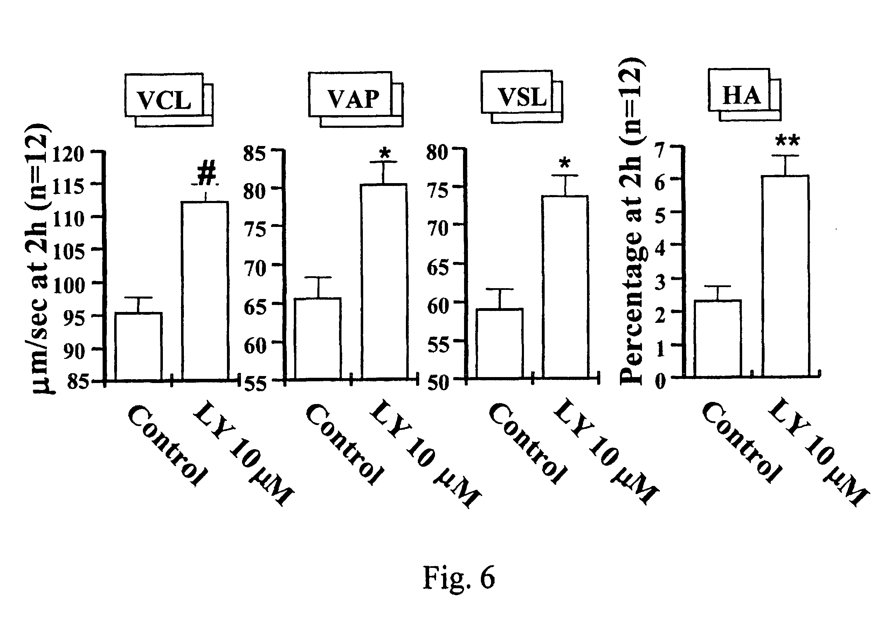

- Fig. 6: shows a histogram depicting the sperm parameters VCL (percentage curvilinear velocity), VAP (average path velocity), VSL (straight-line velocity) and HA (hyperactivated sperm fraction), as determined by computer aided sperm analysis (CASA) in sperm samples from 12 different oligoasthenospermic subjects.

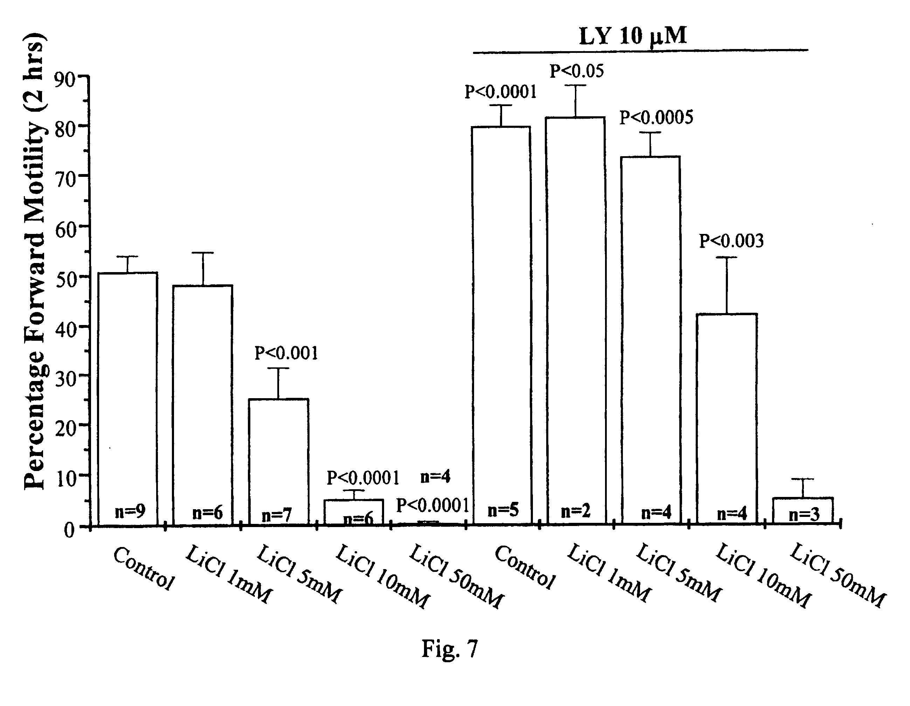

- Fig. 7: shows a histogram depicting the effect of different amounts of Lithiumchloride (LiCl) with and without 10 µM of LY294002 on the percentage forward motility of sperm cells.

### DESCRIPTION OF THE INVENTION

It is an object of the present invention to provide a process for improving the spermatozoa fertilization activity, in particular for increasing the motility of spermatozoa. The process according to the invention comprises treating the spermatozoa with a phosphatidylinositol-3 kinase (PI3K) inhibitor, which is selected from the group consisting of 2-(4-morpholinyl)-8-phenyl-4H-1-benzopyran-4-one (LY294002), and derivatives and analogues thereof.

LY294002 is the chemical compound 2-(4-morpholinyl)-8-phenyl-4H-1-benzopyran-4-one, as described by Vlahos et al. (1994). Derivatives or analogues thereof may also be used according to the invention.

A process wherein the PI3K inhibitor is LY 294002 is highly preferred according to the invention. This agent has been shown to be particularly efficacious for the enhancement of sperm fertilization activity.

The invention is based on the finding that this phosphatidylinositol-3-kinase inhibitor has a pronounced positive effect on parameters determining sperm cell fertilization activity, i.e. the parameters relevant to the capacity of sperm cells to fertilize an oozyte. The most important factors involved in the ability to fertilize are the number of active sperms and the motility of the spermatozoa. According to the WHO manual, motility of 50% is considered the lower limit of normality.

It has now been found in accordance with the invention that the number of motile sperms obtainable from semen samples as well as the motility of the individual spermatozoa can be significantly increased by PI3K inhibitors, which are selected from the group consisting of 2-(4-morpholinyl)-8-phenyl-4H-1-benzopyran-4-one (LY294002), and derivatives and analogues thereof, thus indicating the role of these PI3K inhibitors as enhancers of fertility. This effect is detectable in normospermic individuals. However, it is even more marked in spermatozoa displaying pathogenic features, like oligoasthenospermic patients, i.e. those patients having a reduced total number of spermatozoa and a reduced spermatozoa motility. The invention renders it possible to increase the percentage of spermatozoa with progressive motility, thus significantly improving the probability of successful fertilization. Thus, the process according to the invention helps patients avoid using ICSI in favor of less invasive ART, like conventional IVF.

The terms "spermatozoa" or "sperm (cells)" are used synonymously herein and relate to male gametes. "Semen" or "seminal fluid/liquid" contain sperm cells as well as seminal plasma.

The term "increase of spermatozoa fertilization activity" means any enhancement, improvement, or change to the better of the parameters determining the quality or activity of the sperm cell, such as e.g. percentage curvilinear velocity (VCL), average path velocity (VAP), straight-line velocity (VSL) and hyperactivated sperm fraction (HA). The quality of the spermatozoa determines the fertilization rate in assisted reproduction techniques.

The term "increase" of spermatozoa motility as used herein is meant to encompass any improvement, enhancement, amelioration or change to the better of the quality or fertilization activity or motility or velocity of the cells.

The term "Phosphatidylinositol-3-kinase" or "PI3K" as used herein encompasses any member of the PI3K family, i.e. those related enzymes having the activity outlined in the indroduction.

An "inhibitor of phosphatidylinositol-3-kinase" is also referred to as PI3K and inhibits the production of D-3 phosphoinositides in the cell. The term D-3 phosphoinositides is intended to encompass derivatives of phosphtidylinositol that are phosphorylated in the D-3 position of the inositol ring and comprises, for example, phosphatidylinositol(3)monophosphate (PI(3)P), phosphatidylinositol(3,4)bisphosphate (PI(3,4)P₂) or phosphatidylinositol(3,4,5)trisphosphate (PI(3,4,5)P₃).

In a preferred embodiment, treating the spermatozoa with a phosphatidylinositol-3 kinase (PI3K) inhibitor, which is selected from the group consisting of 2-(4-morpholinyl)-8-phenyl-4H-1-benzopyran-4-one (LY294002), and derivatives and analogues thereof, is performed on the seminal liquid comprising the spermatozoa. Performing the method according to the invention directly on the seminal liquid without any further treatment has the advantage that it is simple and fast. Since the PI3K inhibitor of the invention enhances sperm cell motility, removal of the seminal plasma is not necessary.

In a further preferred embodiment, the process further comprises separating the spermatozoa by spermatozoa separation methods used in assisted reproduction techniques (ART).

Since seminal plasma contains factors that inhibit capacitation and fertilization as well as a considerable amount of non-motile spermatozoa even in a fertile individual, it is advantageous to separate motile sperm cells from fluid, non-motile and morphologically defective spermatozoa. This step is essential in traditional ART like IVF, GIFT or IUl. It leads to an enhancement of the fertilization success rate also in the process according to the invention. It is evident from the examples that the increase in spermatozoa motility by a phosphatidylinositol-3-kinase inhibitor is even more pronounced in spermatozoa which have been separated from the seminal plasma.

In a further preferred embodiment of the invention, separating the spermatozoa is performed by a method selected from the wash and spin method, the sedimentation method, the direct swim-up method, the pellet and swim-up method, and the buoyant density gradient method.

These methods are well known in the art. They are traditionally used in assisted reproduction techniques and described in detail in "A textbook of In vitro Fertilization and Assisted Reproduction, The Bourn Hall guide to clinical and laboratory practice, editor: Peter R. Brinsden, The Parthenon Publishing Group" (1999) on pages 204 to 208. This textbook is referred to hereinafter as the "Bourn Hall guide".

Preferably, separating the spermatozoa is performed by the direct swim-up method.

This method implies self-selection of motile sperms, essentially comprising layering an aliquot of medium on top of a semen sample and allowing it to stand a room temperature for a certain period of time. The motile sperm cells will migrate into the top layer (medium), from which they can be recovered. The method may also include centrifugation step(s).

The advantage of "swim-up" selected spermatozoa is that the motile cells present in the sample are isolated and concentrated and that the proportion of morphologically normal sperm is increased. It is shown in the examples that the process according to the invention leads to an increase the amount of spermatozoa recovered from seminal fluid by the swim-up method. This is due to the increased motility of the sperms, which therefore migrate more quickly and in higher amounts into the upper phase of the sample.

The method may be varied and combined with further isolation/separation techniques, depending on the amount of motile cells in the sample. For example, the swim-up procedure may be performed through the layering of 1 ml of medium containing albumin on a 1 ml of underlying seminal liquid in a test tube. After one hour of incubation at 37°C in the air or in 5% CO₂ the upper phase of the medium to which the spermatozoa with better motility characteristics have migrated is collected. This technique may also comprise or be combined with a centrifugation step, for example centrifugation on Percoll gradients. The separated, isolated or enriched spermatozoa are then used in assisted-reproduction techniques or may be deep-frozen before being further processed, for example.

Advantageously, the incubation of spermatozoa with the PI3K inhibitor is carried out on the seminal fluid, and then swim-up selection is performed. Thereafter, the spermatozoa may be washed one or several times to eliminate the PI3K inhibitor, before being further processed for fertilization.

Preferably, the process according to the invention is performed on mammal spermatozoa, in particular on human spermatozoa.

Preferably, the spermatozoa are treated with an amount of PI3K inhibitor in the range of about 0.01 to 1000 µM, more preferably of about 5 to 500 µM and most preferably of about 10 to 100 µM. Treating the spermatozoa with the PI3K inhibitor advantageously comprises incubating the spermatozoa for a period of time in the range of about 30 minutes to 10 hours, preferably about 1 to 8 hours, most preferably about 2 to 6 hours at a temperature of about 37°C.

It is a further object of the invention to provide spermatozoa having an improved ability of fertilization. Therefore the invention further relates to spermatozoa in which the activity of the phosphatidylinositol-3 kinase is inhibited. The invention also relates to spermatozoa obtainable by the process described above. The spermatozoa in which the phosphatidylinositol-3-kinase is inhibited or which were obtained in a process according to the invention exhibit an improved fertilization activity, a higher motility as compared to untreated sperm cells and thus exhibit a better performance with regard to fertilization.

As mentioned above, sperm cell fertilization activity determines the fertilization rate in ART. The invention therefore further relates to the use of a PI3K inhibitor selected from the group consisting of 2-(4-morpholinyl)-8-phenyl-4H-1-benzopyran-4-one (LY294002), and derivatives and analogues thereof for improving the fertilization rate in assisted reproduction techniques (ART).

Any assisted reproduction method known in the art may be used according to the invention. In preferred embodiments, the assisted reproduction techniques are selected from in vitro fertilization (IVF), gamete intrafallopian transfer (GIFT), and intrauterine insemination (IUI).

The invention further relates to the use of a phosphatidylinositol-3 kinase (PI3K) inhibitor selected from the group consisting of 2-(4-morpholinyl)-8-phenyl-4H-1-benzopyran-4-one (LY294002), and derivatives and analogues thereof for the manufacture of a medicament for the treatment of infertility, in particular male infertility. While the invention is described in more detail for in vitro fertilization techniques, it will be appreciated by the person skilled in the art that the compound may be as efficient in terms of activity when the medicament is administered *in vivo*.

In this case, the medicament is preferably presented in the form of a pharmaceutical composition comprising the phosphatidylinositol-3-kinase inhibitor together with one or more pharmaceutically acceptable carriers and/or excipients.
An "effective amount" refers to an amount of the active ingredients that is sufficient to affect the fertilization activity, in particular the mobility of spermatozoa. The effective amount will depend on the route of administration and the condition of the patient.

"Pharmaceutically acceptable" is meant to encompass any carrier, which does not interfere with the effectiveness of the biological activity of the active ingredient and that is not toxic to the host to which is administered. For example, for parenteral administration, the above active ingredients may be formulated in unit dosage form for injection in vehicles such as saline, dextrose solution, serum albumin and Ringer's solution. Besides the pharmaceutically acceptable carrier, the compositions of the invention can also comprise minor amounts of common additives, such as stabilisers, excipients, buffers and preservatives.

The administration of such active ingredient may be by intravenous, intramuscular or subcutaneous route. Other routes of administration, which may establish the desired blood levels of the respective ingredients, are comprised by the present invention.

The invention further relates to the use of a phosphatidylinositol-3 kinase (PI3K) inhibitor selected from the group consisting of 2-(4-morpholinyl)-8-phenyl-4H-1-benzopyran-4-one (LY294002), and derivatives and analogues thereof, for the manufacture of a medicament for the improvement of spermatozoa fertilization activity, in particular for the increase of spermatozoa motility.

The use of LY294002 as phosphatidylinositol-3-kinase inhibitor is highly preferred.

It is a further object of the present invention to provide for an improvement of ART therapy. The improvement consists in including into known techniques for assisted fertilization a step comprising treating spermatozoa with a phosphatidylinositol-3 kinase (PI3K) inhibitor selected from the group consisting of 2-(4-morpholinyl)-8-phenyl-4H-1-benzopyran-4-one (LY294002), and derivatives and analogues thereof. The further steps used in assisted reproduction techniques are well known to the person skilled in the art and can be taken form the WHO manual (supra) or the Bourn Hall guide (supra).

In a preferred embodiment of the invention, the ART are selected from in vitro fertilization (IVF), gamete intrafallopian transfer (GIFT), or intrauterine insemination (IUI).

It is a further object of the present invention to provide a medium for storage and/or transportation of spermatozoa having improved qualities. The invention therefore also relates to a medium comprising a phosphatidylinositol-3 kinase (PI3K) inhibitor selected from the group consisting of 2-(4-morpholinyl)-8-phenyl-4H-1-benzopyran-4-one (LY294002), and derivatives and analogues thereof. Apart from the PI3K inhibitor, the medium may contain any further component known to be useful for storage and/or transportation, depending on the kind of storage and/or transportation required. For example, the spermatozoa may be stored at room temperature or by cryopreservation. The latter is common for the storage of the cells for a longer period of time. Specific examples of further components of the medium can be taken e.g. from WO 97/16965. Further specific media suitable for cryopreservation of semen are included in Appendix II, pp. 541 and 542 of the Bourn Hall guide (supra), for instance. They could be supplemented with a PI3K inhibitor according to the invention to improve the fertilization activity, in particular the motility of the sperms before fertilization takes place.

In a preferred embodiment, the medium comprises mammal spermatozoa, in particular human spermatozoa. In a highly preferred embodiment, PI3K inhibitor is LY294002.

In yet a further preferred embodiment, the medium according to the invention comprises amounts of the PI3K inhibitor in the range of about 0.01 to 1000 µM, preferably of about 5 to 500 µM, and most preferably of about 10 to 100 µM.

Having now described the invention, it will be more readily understood through reference to the following examples that are provided by way of illustration and are not intended to be limiting the present invention.

### EXAMPLES

In the experiments the following examples are based on, standard methods of in vitro fertilization have been used. With regard to the details of these methods, reference is made to the "WHO manual" (WHO laboratory manual for the examination of human semen and sperm-cervical mucus interactions, 4^{th} edtition, Cambridge University Press 1999). In particular, the direct swim-up method can be taken from pp. 104 to 106 of this manual.

### Example 1: Effect of LY294002 on the rapid motility of spermatozoa

Spermatozoa were prepared according to the standard procedures of IVF. Briefly, spermatozoa were prepared from 3 oligoasthenospermic subjects undergoing semen analysis for couple infertility after informed consent. 10 µM of the PI3K inhibitor LY294002 were added directly to the seminal liquid and incubated for 2 hours for two hours at 37°C and 5% CO₂. The motility of the spermatozoa was then blindly evaluated under the microscope according to WHO manual procedures. The results of this evaluation are shown in Fig. 1. Incubation with the compound causes an increase of rapid motility of the spermatozoa in the seminal liquid (Fig. 1A). Fig. 1 B shows that the increase was observed not only in the rapid mobility (a), but also in the percentage of forward motility (rapid and slow component a + b).

In a group of 7 samples taken from seven individuals, the phosphatidylinositol-3-kinase inhibitor LY294002 was added in a higher concentration (100 µM). After the incubation with the compound for two hours, swim-up selection of the spermatozoa was performed according to procedures described in the WHO-manual. The incubation of the sperm cells with a ten times higher concentration of the inhibitor (100 µM) in combination with the swim-up selection resulted in a significant increase of progressive motility in all of the seven samples, as shown in Fig. 2.

Table I summarizes the results obtained in a similar experiment in 20 oligoasthenospermic patients. The compound was added in a concentration of 10 µM. The effects of LY294002 addition to seminal plasma on the percentage of sperm forward motility both in seminal plasma and in the recovered swim-up fraction measured after incubation with the compound resulted in an increase of 39% of sperm forward motility in the seminal plasma and of 26% in swim-up selected sperm cells.

Table II summarizes the total sperm cell recovery in the swim-up, which was increased by 43% as compared to the control. Thus, incubation of semen with the phosphatidylinositol-3-kinase inhibitor leads to a significant increase in the recovery of spermatozoa during swim-up selection.

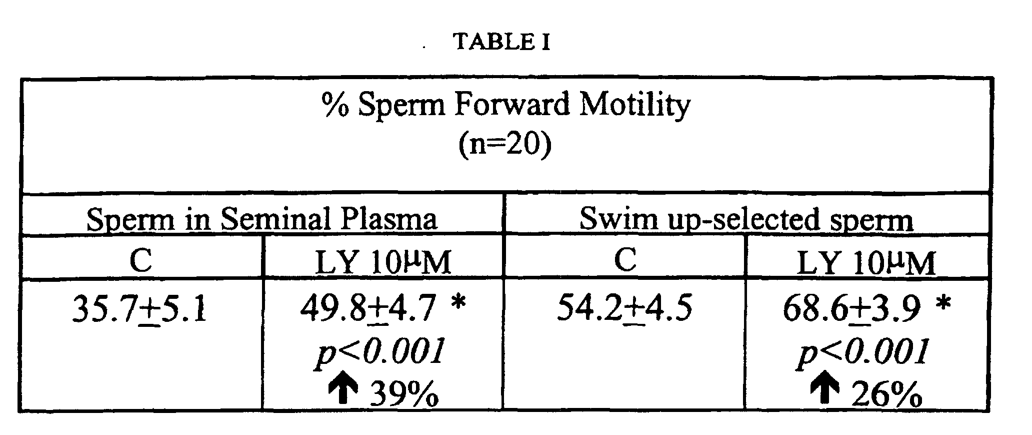

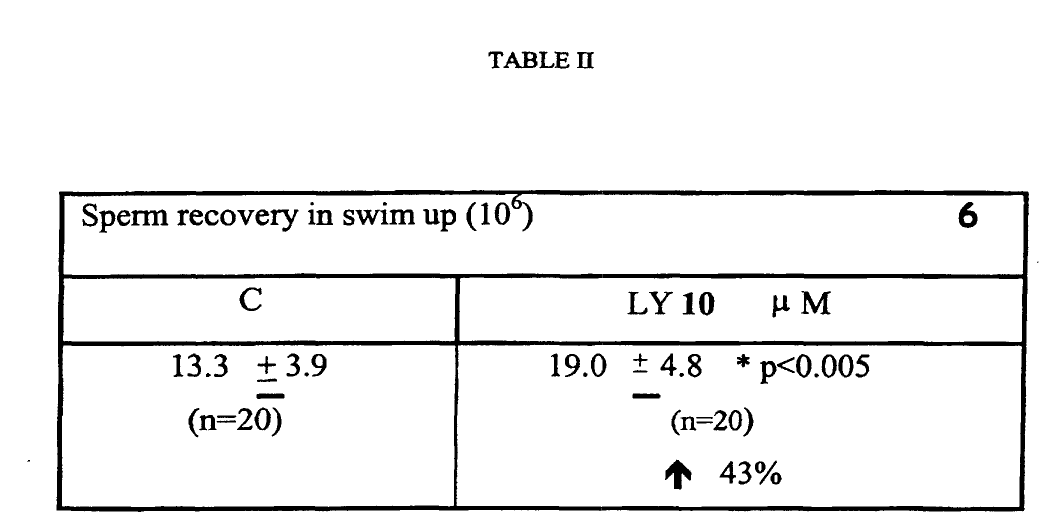

Fig. 3 summarizes the results obtained in a similar experiment on samples from higher numbers of patients. The sperm cells were submitted to the swim-up selection method. Treatment with 10µM of the phosphatidylinositol-3-kinase inhibitor LY294002 resulted in an increase of 58% in the progressive motility (21 patients) as compared to the control (40 patients, without LY294002). Treatment of samples from 29 patients with 100µM of the inhibitor resulted in an increase of the motility of 67% as compared to the control, i.e. in an additional enhancement of spermatozoa motility of about 10%.

The effect of 100µM of LY294002 on the viability of the spermatozoa was also assessed (see insert of Fig. 3). The incubation for two hours with the LY294002 did not alter the vitality of the cells, as evaluated using the eosin test and hypo-osmotic swelling. Even after 48 hours of incubation, no effect on sperm viability was observed in any of the tested samples (not shown).

Fig. 4 shows the results of a further experiment carried out in the same manner as outlined above on samples from 12 individual patients. An improvement of percentage progressive motility after 2 hours of incubation of the sperm cells with LY294002 was clearly detectable, both on the rapid component (a) and on the rapid and slow component (a+b).

The experiments described above were performed on samples from oligoasthenospermic individuals. The effects on the stimulation of progressive motility by LY294002 are more pronounced the more serious the sperm pathology of patients is (oligospermia, asthenospermia, teratospermia). However, the effect of this compound could also be observed in normospermic subjects. The effect of incubation with 10 µM LY 294002 for two hours followed by swim-up selection resulted in an increase from 53.5 ± 2.0 % to 76.3 ± 1.5 % in the froward motility of spermatozoa from 88 randomly selected subjects (not shown).

This effect of LY294002 on samples from normospermic individuals was dose-responsive, as shown in Fig. 5. The values for the rapid forward motility increased with increased doses of the inhibitor applied (from 0 to 1000 µM). The values were calculated with respect to the control (zero) value. The expected sigmoidal curve demonstrates the dose-response effect of the inhibitor on the rapid forward motility of the treated versus untreated spermatozoa, showing an EC50 (effect dose of 50%) of about 1 µM.

Similar effects on spermatozoa have been observed using an unrelated phosphatidylinositol-3-kinase inhibitor, wortmannin.

The experimental evidence described above demonstrates that two different inhibitors of the enzyme phosphatidylinositol-3-kinase are highly efficient in improving spermatozoa motility. This effect is particularly pronounced in pathogenic spermatozoa, but also detectable in normospermic individuals. The results presented above show that incubation of spermatozoa with an phosphatidylinositol-3-kinase inhibitor, if included in traditional assisted reproduction techniques, leads to an increase of sperm cell motility, thus enhancing the probability of fertilization in ART.

### Example 2: Effect of LY294002 on further sperm cell parameters

The increase in forward motility, demonstrated in Example 1, was associated with an increase in sperm parameters related to fertilization activity of the spermatozoa *in vitro,* such as percentage curvilinear velocity (VCL), average path velocity (VAP), straight-line velocity (VSL) and hyperactivated sperm fraction (HA). These parameters were determined by computer aided sperm analysis (CASA) in sperm samples from 12 different oligoasthenospermic subjects. The results are shown in Fig. 6. Each of these parameters were increased in a statistically significant manner by incubation with 10 µM of LY294002 as compared to the control sample, indicating a significant overall improvement of sperm cell fertilization activity.

Similar effects on spermatozoa have been observed using an unrelated phosphatidylinositol-3-kinase inhibitor, wortmannin.
These results demonstrate the overall improvement of the relevant parameters related to sperm cell fertilization activity by phosphatidylinositol-3-kinase inhibitors. Since all of the above parameters are essential for the fertilization rate obtained in assisted fertilization techniques, the invention provides for an agent useful for the treatment of severely impaired male-factor infertility.

### Example 3: Effect of LY294002 on forward motility of H₂O₂ or LiCl treated spermatozoa

It is well known that reactive oxygen species (ROS), which may be generated during sperm preparation for IVF, exert detrimental effects on sperm fertilization potential. In particular, among ROS, H₂O₂ strongly reduces motility when added to sperm samples at micromolar concentrations. Therefore, the effect of LY294002 on H₂O₂ treated sperm cells was evaluated. The compound was added to swim-up selected spermatozoa samples from oligoasthenospermic patients in amounts of 10 µM either alone or in combination with 200 µM of H₂O₂. It was observed that the detrimental effects exerted by 200 µM H₂O₂ on sperm motility were completely reversed by the addition of LY294002 to the incubation medium (Table III), demonstrating a protective activity of the compound with regard to ROS effects.

**TABLE III**

| **Control** **(%)** | **LY294002** **(%)** | **H**_{**2**}**O**_{**2**} **(%)** | **LY294002+H**_{**2**}**O**_{**2**} **(%)** |
|---|---|---|---|
| 45.4±5.0 (n=12) | 71.7±3.2** (n=12) | 26.5±4.9** (n=12) | 52.6±5.5* (n=12) |

| | | | |
|---|---|---|---|
| *P<0.002 vs H₂O₂; | | | |
| **P<0.0001 vs Control | | | |

LiCl is also a known inhibitor of sperm cell motility. An incubation of swim-up selected spermatozoa with 10 µM LY294002 for two hours either with or without different concentrations of LiCI resulted in reversing the effect of LiCl induced inhibition of sperm motility (Fig. 7)
Similar effects on spermatozoa have been observed using an unrelated phosphatidylinositol-3-kinase inhibitor, wortmannin.

In this example, the activity of phosphatidylinositol-3-kinase inhibitors to rescue spermatozoa from deleterious agents, which may be generated in assisted fertilization techniques has been demonstrated. Therefore, the invention provides for a major improvement of ART, leading to a higher fertilization rate and eliminating some of the most serious drawbacks of these techniques.

### REFERENCES

Edwards RG "Widening perspectives of intracytoplasmic sperm injection" (1999). Nature Medicine 5, 377-378.
Luetjens CM, Payne C, Schatten G "Non-random chromosome positioning in human sperm and sex chromosome anomalies intracytoplasmatic sperm injection (1999). The Lancet 353, 1240.
Vanhaesebroeck B, Leevers SJ, Panayotou G, Waterfield MD "Phosphatidylinositol-3-kinases: a conserved family of signal transducers" (1997). Trends. Biochem. Sci. 22, 267-72.
Vlahos CJ, Matter WF, Brown RF, Traynor Kaplan AE, Heyworth PG, Prossnitz ER "Investigation of neutrophil signal transduction using a specific inhibitor of phosphatidylinositol-3-kinase" (1995). J. Immunol. 154, 2413-22.
Vlahos CJ, Matter WF, Hui KY, Brown RF "A specific inhibitor of phosphatidylinositol-3-kinase, 2-(4-morpholinyl)-8-pheny-4H-1-benzopyran-4-one (LY294002)" (1994). J. Biol. Chem. 269, 5241-48.

## Claims

1. In vitro process for improving the fertilization activity of spermatozoa, in particular for increasing spermatozoa motility, comprising treating the spermatozoa in vitro with a phosphatidylinositol-3 kinase (PI3K) inhibitor, which is selected from the group consisting of 2-(4-morpholinyl)-8-phenyl-4H-1-benzopyran-4-one (LY294002), and derivatives and analogues thereof.

2. Process according to claim 1, wherein treating the spermatozoa with the phosphatidylinositol-3 kinase (PI3K) inhibitor is performed on seminal liquid comprising the spermatozoa.

3. Process according to claim 1 or 2, further comprising separating the spermatozoa by spermatozoa separation methods used in assisted reproduction techniques (ART).

4. Process according to claim 3, wherein separating the spermatozoa is performed by a method selected from the wash and spin method, the sedimentation method, the direct swim-up method, the pellet and swim-up method, the buoyant density gradient method.

5. Process according to claim 4, wherein separating the spermatozoa is performed by the direct swim-up method.

6. Process according to any of claims 1 to 5, wherein the process is performed on mammal spermatozoa, in particular on human spermatozoa.

7. Process according to any of claims 1 to 6, wherein spermatozoa are treated with an amount of PI3K inhibitor in the range of about 0.01 to 1000 µM, about 5 to 500 µM, or about 10 to 100 µM.

8. Process according to any of claims 1 to 7, wherein treating the spermatozoa with the PI3K inhibitor comprises incubating the spermatozoa for a period of time in the range of about 30 minutes to 10 hours or about 1 to 8 hours or about 2 to 6 hours at a temperature of around 37°C.

9. Spermatozoa obtainable by the process according to any of claims 1 to 8.

10. Use of a PI3K inhibitor selected from the group consisting of 2-(4-morpholinyl)-8-phenyl-4H-1-benzopyran-4-one (LY294002), and derivatives and analogues thereof, for improving in vitro the fertilization rate in assisted reproduction techniques (ART).

11. Use according to claim 10, wherein the assisted reproduction techniques are selected from in vitro fertilization (IVF), gamete intrafallopian transfer (GIFT), or intrauterine insemination (IUI).

12. Use of a phosphatidylinositol-3 kinase (PI3K) inhibitor selected from the group consisting of 2-(4-morpholinyl)-8-phenyl-4H-1-benzopyran-4-one (LY294002), and derivatives and analogues thereof, for the manufacture of a medicament for the treatment of infertility, in particular male infertility.

13. Use of a phosphatidylinositol-3 kinase (PI3K) inhibitor selected from the group consisting of 2-(4-morpholinyl)-8-phenyl-4H-1-benzopyran-4-one (LY294002), and derivatives and analogues thereof, for the manufacture of a medicament for improving spermatozoa fertilization activity, in particular for increasing spermatozoa motility.

14. Method of treating spermatozoa 'n vitro for ART therapy with a phosphatidylinositol-3 kinase (PI3K) inhibitor selected from the group consisting of 2-(4-morpholinyl)-8-phenyl-4H-1-benzopyran-4-one (LY294002), and derivatives and analogues thereof.

15. Method according to claim 14, wherein the ART are selected from in vitro fertilization (IVF), gamete intrafallopian transfer (GIFT), or intrauterine insemination (IUI).

16. A medium for storage and/or transportation of spermatozoa comprising a phosphatidylinositol-3 kinase (PI3K) inhibitor, wherein the PI3K inhibitor is selected from the group consisting of 2-(4-morpholinyl)-8-phenyl-4H-1-benzopyran-4-one (LY294002), and derivatives and analogues thereof.

17. Medium according to claim 17, for the storage and/or transportation of mammal spermatozoa, in particular human spermatozoa.

18. Medium according to any of claims 17 or 18, comprising an amount of PI3K inhibitor in the range of about 0.01 to 1000 µM, about 5 to 500 µM, or about 10 to 100 µM.

## Patentansprüche

1. In-Vitro-Verfahren zur Verbesserung der Fertilisationsaktivität von Spermatozoen, insbesondere zur Steigerung der Spermatozoen-Motilität, umfassend das Behandeln der Spermatozoen *in vitro* mit einem Phosphatidylinositol-3-Kinase(PI3K)-Inhibitor, welcher ausgewählt ist aus der Gruppe, bestehend aus 2-(4-Morpholinyl)-8-phenyl-4H-1-benzopyran-4-on (LY294002), und Derivaten und Analogen davon.

2. Verfahren nach Anspruch 1, wobei das Behandeln der Spermatozoen mit dem Phosphatidylinositol-3-Kinase(PI3K)-Inhibitor auf Samenflüssigkeit, umfassend die Spermatozoen, durchgeführt wird.

3. Verfahren nach Anspruch 1 oder 2, weiter umfassend das Abtrennen der Spermatozoen durch Spermatozoen-Abtrennungsmethoden, verwendet bei assistierten Reproduktionstechniken (ART).

4. Verfahren nach Anspruch 3, wobei das Abtrennen der Spermatozoen durch eine Methode, ausgewählt aus der "wash und spin-Methode", der Sedimentationsmethode, der direkten "swim-up-Methode", der "Pellet-und-swim-up-Methode" und der Auftriebsdichtegradientenmethode, durchgeführt wird.

5. Verfahren nach Anspruch 4, wobei das Abtrennen der Spermatozoen durch die direkte "swim-up-Methode" durchgeführt wird.

6. Verfahren nach einem der Ansprüche 1 bis 5, wobei das Verfahren an Säugetier-Spermatozoen, insbesondere an humanen Spermatozoen, durchgeführt wird.

7. Verfahren nach einem der Ansprüche 1 bis 6, wobei die Spermatozoen mit einer Menge des PI3K-Inhibitors im Bereich von ungefähr 0,01 bis 1000 µM, ungefähr 5 bis 500 µM oder ungefähr 10 bis 100 µM behandelt werden.

8. Verfahren nach einem der Ansprüche 1 bis 7, wobei das Behandeln der Spermatozoen mit dem PI3K-Inhibitor das Inkubieren der Spermatozoen für einen Zeitraum im Bereich von ungefähr 30 Minuten bis 10 Stunden oder ungefähr 1 bis 8 Stunden oder ungefähr 2 bis 6 Stunden bei einer Temperatur von ungefähr 37°C umfaßt.

9. Spermatozoen, erhältlich durch das Verfahren nach einem der Ansprüche 1 bis 8.

10. Verwendung eines PI3K-Inhibitors, ausgewählt aus der Gruppe, bestehend aus 2-(4-Morpholinyl)-8-phenyl-4H-1-benzopyran-4-on (LY294002), und Derivaten und Analogen davon, zur Verbesserung in vitro der Fertilisationsrate bei assistierten Reproduktionstechniken (ART).

11. Verwendung nach Anspruch 10, wobei die assistierten Reproduktionstechniken ausgewählt sind aus der In-Vitro-Fertilisation (IVF), des Intra-Eileiter-Transfers von Gameten (GIFT) oder der Intrauterinen Insemination (IUI).

12. Verwendung eines Phosphatidylinositol-3-Kinase(PI3K)-Inhibitors, ausgewählt aus der Gruppe, bestehend aus 2-(4-Morpholinyl)-8-phenyl-4H-1-benzopyran-4-on (LY294002), und Derivaten und Analogen davon, zur Herstellung eines Medikaments zur Behandlung der Infertilität, insbesondere der männlichen Infertilität.

13. Verwendung eines Phosphatidylinositol-3-Kinase(PI3K)-Inhibitors, ausgewählt aus der Gruppe, bestehend aus 2-(4-Morpholinyl)-8-phenyl-4H-1-benzopyran-4-on (LY294002), und Derivaten und Analogen davon, zur Herstellung eines Medikaments zur Verbesserung der Fertilisationsaktivität von Spermatozoen, insbesondere zur Steigerung der Spermatozoenmotilität.

14. Verfahren zum Behandeln von Spermatozoen in vitro zur ART-Therapie mit einem Phosphatidylinositol-3-Kinase(PI3K)-Inhibitor, ausgewählt aus der Gruppe, bestehend aus 2-(4-Morpholinyl)-8-phenyl-4H-1-benzopyran-4-on (LY294002), und Derivaten und Analogen davon.

15. Verfahren nach Anspruch 14, wobei die ART aus der In-Vitro-Fertilisation (IVF), dem Intra-Eileiter-Transfer von Gameten (GIFT) oder der Intrauterinen Insemination (IUI) ausgewählt sind.

16. Medium zur Aufbewahrung und/oder zum Transport von Spermatozoen, umfassend einen Phosphatidylinositol-3-Kinase(PI3K)-Inhibitor, wobei der PI3K-Inhibitor ausgewählt ist aus der Gruppe, bestehend aus 2-(4-Morpholinyl)-8-phenyl-4H-1-benzopyran-4-on (LY294002), und Derivaten und Analogen davon.

17. Medium nach Anspruch 17 zur Aufbewahrung und/oder zum Transport von Säugetier-Spermatozoen, insbesondere humanen Spermatozoen.

18. Medium nach einem der Ansprüche 17 oder 18, umfassend eine Menge von PI3K-Inhibitor im Bereich von ungefähr 0,01 bis 1000 µM, ungefähr 5 bis 500 µM oder ungefähr 10 bis 100 µM.

## Revendications

1. Procédé *in vitro* pour améliorer l'activité de fertilisation des spermatozoïdes en particulier pour augmenter la mobilité des spermatozoïdes, comprenant le fait de traiter les spermatozoïdes *in vitro* avec un inhibiteur de phosphatidylinositol-3 kinase (PI3K) choisi dans le groupe constitué du 2-(4-morpholinyl)-8-phényl-4H-1-benzopyran-4-one (LY294002) et les dérivés et analogues de celui-ci.

2. Procédé selon la revendication 1, dans lequel le fait de traiter les spermatozoïdes avec un inhibiteur de phosphatidylinositol-3 kinase (PI3K) est effectué sur le liquide séminal comprenant les spermatozoïdes.

3. Procédé selon la revendication 1 ou 2, comprenant de plus le fait de séparer les spermatozoïdes par des méthodes de séparation de spermatozoïdes utilisées dans les techniques de reproduction assistée (TRA).

4. Procédé selon la revendication 3, dans lequel la séparation des spermatozoïdes est effectuée par une méthode choisie parmi la méthode de lavage et centrifugation, la méthode de sédimentation, la méthode directe au surnageant, la méthode au culot et surnageant, ou la méthode de flottation en gradient de densité.

5. Procédé selon la revendication 4, dans lequel le fait de séparer les spermatozoïdes est effectué par la méthode directe en surnageant.

6. Procédé selon l'une quelconque des revendications 1 à 5, dans lequel le procédé est effectué sur des spermatozoïdes de mammifère, en particulier sur des spermatozoïdes humains.

7. Procédé selon l'une quelconque des revendications 1 à 6, dans lequel les spermatozoïdes sont traités avec une quantité d'inhibiteurs de PI3K de 0,01 à 1000 µM, à peu près de 5 à 500 µM, ou à peu près de 10 à 100 µM.

8. Procédé selon l'une quelconque des revendications 1 à 7, dans lequel le fait de traiter les spermatozoïdes avec un inhibiteur de PI3K comprend l'incubation des spermatozoïdes pendant une période de temps d'à peu près 30 minutes à 10 heures ou à peu près 1 à 8 heures, ou à peu près 2 à 6 heures à une température d'à peu près 37 °C.

9. Spermatozoïdes susceptibles d'être obtenus par le procédé selon l'une quelconque des revendications 1 à 8.

10. Utilisation d'un inhibiteur de PI3K choisi dans le groupe constitué du 2-(4-morpholinyl)-8-phényl-4H-1-benzopyran-4-one (LY294002), et des dérivés et analogues de celui-ci, pour l'amélioration *in vitro* du taux de fertilisation dans les techniques de reproduction assistée (TRA).

11. Utilisation selon la revendication 10, dans laquelle les techniques de reproduction assistée sont choisies parmi la fertilisation *in vitro* (FIV), le transfert de gamète intrafallopien (TGIF) ou l'insémination intra-utérine (IIU).

12. Utilisation d'un inhibiteur de la phosphatidylinositol-3 kinase (PI3K) choisi dans le groupe constitué du 2-(4-morpholinyl)-8-phényl-4H-1-benzopyran-4-one (LY294002), et des dérivés et analogues de celui-ci, pour la fabrication d'un médicament pour le traitement de l'infertilité, en particulier l'infertilité mâle.

13. Utilisation d'un inhibiteur de la phosphatidylinositol-3 kinase (PI3K) choisi dans le groupe constitué de 2-(4-morpholinyl)-8-phényl-4H-1-benzopyran-4-one (LY294002), et des dérivés et analogues de celui-ci, pour la fabrication d'un médicament pour l'amélioration de l'activité de fertilisation des spermatozoïdes, en particulier pour augmenter la mobilité des spermatozoïdes.

14. Procédé de traitement des spermatozoïdes *in vitro* pour une thérapie par les techniques de reproduction assistée, par un inhibiteur de la phosphatidylinositol-3 kinase (PI3K) choisi dans le groupe constitué de 2-(4-morpholinyl)-8-phényl-4H-1-benzopyran-4-one (LY294002), et ses dérivés et analogues.

15. Procédé selon la revendication 14, dans lequel les techniques de reproduction assistée sont choisies parmi la fertilisation *in vitro* (FIV), le transfert de gamète intrafallopien (TGIF) ou l'insémination intra-utérine (IIU).

16. Milieu de stockage et/ou de transport des spermatozoïdes comprenant un inhibiteur de la phosphatidylinositol-3 kinase (PI3K) dans lequel l'inhibiteur de PI3K est choisi dans le groupe constitué de 2-(4-morpholinyl)-8-phényl-4H-1-benzopyran-4-one (LY294002), et les dérivés et analogues de celui-ci.

17. Milieu selon la revendication 16, pour le stockage et le transport des spermatozoïdes de mammifère, en particulier des spermatozoïdes humains.

18. Milieu selon la revendication 16 ou 17, comprenant une quantité d'inhibiteur de PI3K d'à peu près 0,01 à 1000 µM, à peu près 5 à 500 µM, ou à peu près 10 à 100 µM.
